# EUROPÄISCHE PATENTSCHRIFT

(11) **EP 0 868 125 B1**
(45) Veröffentlichungstag und Bekanntmachung des Hinweises auf die Patenterteilung: **31.10.2001**
(21) Anmeldenummer: 96944004.9
(22) Anmeldetag: 19.12.1996
(51) Int. Cl.: A23L 1/304, B01J 20/24

(54) **ADSORBENS FÜR PHOSPHAT AUS WÄSSRIGEM MEDIUM, DESSEN HERSTELLUNG UND VERWENDUNG**
ADSORBENT FOR PHOSPHATE FROM AN AQUEOUS MEDIUM, PRODUCTION AND USE OF SAID ADSORBENT
ADSORBANTS POUR PHOSPHATES FORMES A PARTIR D'AGENTS AQUEUX, LEUR PRODUCTION ET LEUR UTILISATION

(30) Priorität: 19.12.1995 DE 19547356
(43) Veröffentlichungstag der Anmeldung: 07.10.1998
(73) Patentinhaber: VIFOR (INTERNATIONAL) AG, 9001 St Gallen (CH)
(72) Erfinder: GEISSER, Peter, CH-9014 St. Gallen (CH); PHILIPP, Erik, CH-9014 St. Gallen (CH)
(74) Vertreter: Hrabal, Ulrich, Dr.
(86) Internationale Anmeldenummer: EP9605695
(87) Internationale Veröffentlichungsnummer: WO9722266

(56) Entgegenhaltungen:
- EP-A- 0 052 206
- EP-A- 0 164 657
- WO-A-95/22908
- AT-A- 382 326
- DE-A- 4 239 442
- FR-A- 2 387 045

## Beschreibung

Die Erfindung betrifft ein Adsorbens für Phosphat aus wäßrigem Medium, beispielsweise aus wäßrigen Lösungen. Es ist insbesondere als Adsorbens für anorganisches und nahrungsmittelgebundenes Phosphat geeignet; insbesondere als Präparat zur oralen Anwendung für die Prophylaxe und die Behandlung des hyperphosphatämischen Zustands.

Bei chronisch niereninsuffizienten Patienten treten bedingt durch die Abnahme der glomulären Filtrationsrate pathologisch erhöhte Serumphosphatspiegel auf. Der damit einhergehende sekundäre Hyperparathyreoidismus muß als eine der Ursachen für die Entstehung einer renalen Osteopathie betrachtet werden. Normalerweise versucht man, die Phosphatbilanz durch Dialyse, Gabe oraler Phosphatadsorber, welche die Resorption der Nahrungsphosphate im Gastrointestinaltrakt verhindern sollen, oder die Kombination beider Verfahren im Gleichgewicht zu halten, wobei dies nach dem heutigen Stand der Technik entweder zu wenig effizient, nicht kostengünstig oder mit Nebenwirkungen behaftet ist.

So verursachen die als orale Phosphatadsorber eingesetzten Aluminium-III-salze neben dem Dialyse-Enzephalopathie-Syndrom renale Osteopathie und mikrozytäre Anämie. Bei Verwendung von Calciumsalzen treten häufig Hypercalcämien in Verbindung mit Gefäß- und Weichteilverkalkungen sowie gastrointestinale Beschwerden auf (Dialyse Journal 37, 1-40 (1991).

Außerdem wurde in der DE 42 39 442 A1 die Verwendung von mit polynuklearen Metalloxidhydroxiden modifizierten Adsorptionsmaterialien vorgeschlagen, wobei die Verwendung der dort beschriebenen wasserlöslichen Eisendextrane und -dextrine den Nachteil besitzt, resorbierbar zu sein. Durch Verwendung vernetzter Polysaccaridträger können Komplexe hergestellt werden, die wenig Eisen freisetzen. Der Nachteil dieser Komplexe, die auf alpha-Eisenhydroxiden basieren, liegt einerseits in den hohen Kosten der zu verwendenden vernetzten Polysaccharidträger und andererseits in dem verbesserungsbedürftigen Phosphatadsorptionsvermögen.

Aufgabe der vorliegenden Erfindung ist daher die Bereitstellung von Adsorbentien für Phosphat aus wäßrigem Medium, insbesondere aus wäßriger Lösung. Die Adsorbentien sollen insbesondere für anorganisches und nahrungsmittelgebundenes Phosphat aus Körperflüssigkeiten, Speisebrei und Nahrungsmitteln geeignet sein, ein verbessertes Phosphatadsorptionsvermögen aufweisen und einfach und kostengünstig herstellbar sein.

Es hat sich gezeigt, daß diese Aufgabe gelöst werden kann durch die einen Gegenstand der Erfindung bildenden Adsorbentien für Phosphat aus wäßrigem Medium, die durch Kohlenhydrate und/oder Humussäure stabilisiertes polynukleares beta-Eisenhydroxid enthalten. Die Adsorbentien sind insbesondere geeignet für die Adsorption von Phosphaten aus wäßrigen Lösungen, beispielsweise für die Adsorption von anorganischen und nahrungsmittelgebundenen Phosphaten.

Im Rahmen der vorliegenden Erfindung hat es sich gezeigt, daß polynukleares beta-Eisenhydroxid ein überlegenes Phosphatadsorptionsvermögen besitzt. Da polynukleares beta-Eisenhydroxid jedoch unter Strukturänderung altert, konnte es bisher für diesen Zweck nicht eingesetzt werden. Im Rahmen der Erfindung wurde gefunden, daß eine Stabilisierung durch geeignete Verbindungen, insbesondere Kohlenhydrate und Humussäure erfolgen kann. Ohne an eine Theorie gebunden zu sein, wird davon ausgegangen, daß jedoch keine Komplexbildung mit den Kohlenhydraten stattfindet.

Einen weiteren Gegenstand der Erfindung bildet das Verfahren zur Herstellung des durch Kohlenhydrate und/oder Humussäure stabilisierten polynuklearen beta-Eisenhydroxids, das als Adsorbens oder in Adsorbentien für anorganisches und nahrungsmittelgebundenes Phosphat eingesetzt werden kann. Bei dem erfindungsgemäßen Verfahren wird eine wäßrige Basenlösung mit einer wäßrigen, Chloridionen enthaltenden Lösung von Eisen-III-salz unter Bildung einer Suspension von braunem beta-Eisenhydroxid mit einem pH-Wert von 3 bis 10, vermischt. Man läßt die Suspension anschließend ruhen. In der Praxis kann die Suspension stehengelassen werden, beispielsweise während 1 bis 5 Stunden. Gelegentlich kann dabei kurz gerührt werden, beispielsweise kann in Abständen von jeweils 10 Minuten kurz (beispielsweise 10 minütig) gerührt werden. Das erhaltene beta-Eisenhydroxid wird mit Wasser gewaschen. Dies kann beispielsweise durch Dekantieren, Filtrieren und/oder Zentrifugieren erfolgen. Die Wäsche wird solange durchgeführt, bis störende Anionen, beispielsweise Chloridionen entfernt sind. Man erhält ein feuchtes Produkt; dieses wird nicht zur Trocknung gebracht. Das feuchte Produkt wird in Wasser aufgeschlämmt. Die Wassermenge ist nicht kritisch; es wird so gearbeitet, daß der Eisengehalt der erhaltenen Suspension (berechnet als Fe) bis zu 6 Gewichtsprozent, besonders bevorzugt 2 bis 6 Gewichtsprozent beträgt. Anschließend erfolgt die Zugabe von einem oder mehreren Kohlenhydraten und/oder Humussäure in einer derartigen Menge, daß der erhaltene Feststoff maximal 40 Gew.-% Eisen enthält.

Als Alkalicarbonat- oder Alkalibicarbonatlösung kann beispielsweise Natriumcarbonat bzw. Natriumbicarbonat in wäßriger Lösung eingesetzt werden.

Als Eisen-III-salz können wasserlösliche Salze von beispielsweise anorganischen oder organischen Säuren eingesetzt werden; bevorzugt ist Eisen-III-chlorid.

Die Herstellung des β-Eisenhydroxids (Akaganeit) ist grundsätzlich im Stand der Technik bekannt und beispielsweise in der Literaturstelle U. Schwertmann, R. M. Cornell "Iron Oxides in the Laboratory", VCH Verlagsgesellschaft mbH, 1991, Seiten 95 - 100 beschrieben. Aus wirtschaftlichen Gründen kann es bei der industriellen Herstellung des β-Eisenhydroxids enthaltenden Adsorptionsmittels erforderlich sein, das Eisen quantitativ bei höheren pH-Werten auszufällen. Wie in der zuvor genannten Literaturstelle auf Seite 100 beschrieben wird, wird dabei auch Ferrihydrit ausgefällt. Solche Mischungen von β-Eisenhydroxid und Ferrihydrit können in der vorliegenden Erfindung ebenfalls als Adsorptionsmittel verwendet werden.

Zu der vorstehend erhaltenen Suspension werden ein oder mehrere Kohlenhydrate und/oder Humussäure gefügt. Bevorzugt werden wasserlösliche Verbindungen eingesetzt. Die Kohlenhydrate und/oder Humussäure können in fester Form zugesetzt werden, wobei sie sich in dem vorhandenen Wasser lösen können. Es ist jedoch auch möglich, wäßrige Lösungen von Kohlenhydraten zuzufügen.

Es ist bevorzugt, die Menge an Kohlenhydraten bzw. Humussäure so zu wählen, daß mindestens 0,5 g Kohlenhydrat oder Humussäure pro g Eisen (berechnet als Fe) zugesetzt werden. Der Eisengehalt beträgt maximal 40 Gewichtsprozent. Der maximale Gehalt an Kohlenhydraten und/oder Humussäure unterliegt keiner Begrenzung und wird in erster Linie durch wirtschaftliche Gründe bestimmt.

Als Kohlenhydrate können insbesondere lösliche Kohlenhydrate eingesetzt werden, wie verschiedene Zucker, z.B. Agarose, Dextran, Dextrin, Dextranderivate, Zellulose und Zellulosederivate, Saccharose, Maltose, Lactose oder Mannit.

Die erfindungsgemäß bereitgestellten Adsorptionsmaterialien aus unlöslichem stabilisiertem polynuklearem beta-Eisenhydroxid haben den Vorteil, daß sie neben einem hohen Phosphatbindungsvermögen wenig Eisen freisetzen und kostengünstig herzustellen sind.

Es kann zweckmäßig und bevorzugt sein, den erfindungsgemäßen Adsorbentien ein oder mehrere Calciumsalze zuzusetzen. Als Calciumsalze kommen beispielsweise Salze anorganischer oder organischer Säuren, insbesondere Calciumacetat, in Frage. Durch die Zugabe des Calciumsalzes wird das Phosphatbindungsvermögen erhöht, insbesondere bei höheren pH-Werten. Es ist besonders günstig, solche mit Calciumsalzen versehene Adsorbentien bei pH-Werten von mehr als 5 einzusetzen, da selbst dann das vollständige Phosphatbindungsvermögen beibehalten bleibt.

Es hat sich gezeigt, daß eine Zugabe von 400 mg bis 2 g, beispielsweise etwa 1 g Calciumsalz, insbesondere Calciumacetat pro g Eisen besonders günstig ist.

Die erfindungsgemäßen Adsorbentien können beispielsweise zur oralen Anwendung formuliert sein. Sie können als solche oder zusammen mit üblichen Arzneimittelzusätzen, wie üblichen Trägern oder Hilfsmitteln, formuliert werden. Beispielsweise kann eine Einkapselung erfolgen, wobei als Einkapselungsmittel übliche auf dem pharmazeutischen Sektor verwendete Materialien eingesetzt werden können. Es ist auch möglich, die Adsorbentien gegebenenfalls zusammen mit Hilfs- und Zusatzstoffen als Granulate, Tabletten, Dragees oder beispielsweise in Sachets abgefüllt, bereitzustellen. Die Tagesdosis der erfindungsgemäßen Adsorbentien liegt beispielsweise bei 1 bis 3 g, vorzugsweise bei etwa 1,5 g Eisen.

Die erfindungsgemäßen Adsorbentien können zur Adsorption von anorganischen oder nahrungsmittelgebundenem Phosphat aus Körperflüssigkeiten oder aus Magen-Darminhalt, insbesondere bei gleichzeitiger Aufnahme von Nahrung verwendet werden. Zur Verwendung zur Adsorption von nahrungsmittelgebundenem Phosphat können sie beispielsweise Nahrungsmitteln beigemischt werden. Hierzu können beispielsweise Formulierungen erstellt werden, wie sie vorstehend für Arzneimittel beschrieben wurden.

Die Erfindung wird durch die folgenden Beispiele näher erläutert:

### Beispiel 1

Zu 241,5 g Sodalösung (d²⁰ = 1,185 g/ml) werden im Verlauf von 30 min. unter Rühren (Flügelrührer) 275 g Eisen(III)-chloridlösung (d²⁰ = 1,098 g/ml) zugetropft. Die Suspension wird 2 Stunden stehengelassen. Während dieser Zeit wird sechsmal 10 min. gerührt. Die entstandene Suspension wird nun unter Rühren mit 300 ml Wasser versetzt, 1 Stunde stehengelassen und dann die überstehende Flüssigkeit abdekantiert. Dieser Vorgang wird fünfmal wiederholt.

Man erhält 208,3 g einer Suspension mit einem Eisengehalt von 4,8 (komplexometrisch ermittelt).

Zu den 208,3 g obiger Suspension werden je 15 g Saccharose und Stärke zugegeben. Die Suspension wird sodann am Rotationsverdampfer bei 50°C eingeengt und im Hochvakuum bei 40°C getrocknet.

Man erhält 47,2 g Pulver mit einem Eisengehalt von 21,2 % (komplexometrisch ermittelt).

### Beispiel 2

Bestimmung der Bindungskapazität des gemäß Beispiel 1 hergestellten Materials für anorganisches Phosphat aus einer wäßrigen Phosphatlösung:

Zu 236 mg des gemäß Beispiel 1 hergestellten Materials (entsprechend 0,9 mmol Eisen) werden 10 ml Natriumphosphatlösung (13,68 g/l Na₃PO₄ x 12 H₂O) zugegeben (Molverhältnis Fe : P = 1 : 0,4). Nach Einstellen des pH-Wertes von 3,0, 5,5 oder 8,0 läßt man die Suspension 2 Stunden bei 37°C reagieren. Danach wird die Suspension zentrifugiert, der Überstand abdekantiert, mit destilliertem Wasser auf 25 ml aufgefüllt und dessen Phosphorgehalt mittels eines Phosphor-Molybdän-Tests photometrisch bestimmt.

**Tabelle 1**

| Phosphatbindungsvermögen, Molverhältnis Fe : P = 1 : 0,4 | | | | | |
|---|---|---|---|---|---|
| pH | mg Phosphor in der überstehenden Lösung | mg Phosphat in der überstehenden Lösung | mg Phosphor adsorbiert | mg Phosphat adsorbiert | Adsorption in % |
| 3,0 | - | - | 11,16 | 28,43 | 100 |
| 5,5 | 0,78 | 1,99 | 10,38 | 26,44 | 93 |
| 8,0 | 2,9 | 7,39 | 8,26 | 21,04 | 74 |

### Beispiel 3

Bestimmung der Bindungskapazität des gemäß Beispiel 1 hergestellten Materials für anorganisches Phosphat aus einer wäßrigen Phosphatlösung analog Beispiel 2, jedoch unter Zugabe von 10 ml Natriumphosphatlösung mit 27,36 g/l Na₃PO₄ x 12 H₂O zu 236 mg des oben genannten Materials (Molverhältnis Fe : P = 1 : 0,8):

**Tabelle 2**

| Phosphatbindungsvermögen, Molverhältnis Fe : P = 1 : 0,8 | | | | | |
|---|---|---|---|---|---|
| pH | mg Phosphor in der überstehenden Lösung | mg Phosphat in der überstehenden Lösung | mg Phosphor adsorbiert | mg Phosphat adsorbiert | Adsorption in % |
| 3,0 | 1,67 | 4,26 | 9,49 | 24,17 | 85 |
| 5,5 | 2,57 | 6,54 | 8,59 | 21,89 | 77 |
| 8,0 | 4,24 | 10,8 | 6,92 | 17,63 | 62 |

### Beispiel 4

Bestimmung der Bindungskapazität des gemäß Beispiel 1 hergestellten Materials für organisches Phosphat aus einer wäßrigen Phosphatlösung.

Zu 236 mg des gemäß Beispiel 1 hergestellten Materials (entspr. 0,9 mmol Eisen) werden 10 ml Glycerophosphatlösung (11,02 g/l Glycerophosphat Dinatriumsalz Pentahydrat) zugegeben (Molverhältnis Fe : P = 1 : 0,4). Nach Einstellen des pH-Wertes von 3,0, 5,5 oder 8,0 läßt man die Suspension 2 Stunden bei 37°C reagieren. Danach wird die Suspension zentrifugiert, der Überstand abdekantiert, mit destilliertem Wasser auf 25 ml aufgefüllt und dessen Phosphorgehalt nach Aufschluß des organisch gebundenen Phosphats und Fällung des anorganischen Phosphats mittels Lorenz-Reagens gravimetrisch bestimmt.

**Tabelle 3:**

| Phosphatbindungsvermögen, Molverhältnis Fe : P = 1 : 0,4 | | | | | |
|---|---|---|---|---|---|
| pH | mg Phosphor in der überstehenden Lösung | mg Phosphat in der überstehenden Lösung | mg Phosphor adsorbiert | mg Phosphat adsorbiert | Adsorption in % |
| 3,0 | 5,69 | 14,50 | 5,47 | 13,93 | 49 |
| 5,5 | 6,70 | 17,06 | 4,46 | 11,37 | 40 |
| 8,0 | 7,81 | 19,90 | 3,35 | 8,53 | 30 |

### Beispiel 5

Bestimmung der Bindungskapazität des gemäß Beispiel 1 hergestellten Materials für organisches Phosphat aus einer wäßrigen Phosphatlösung:

Zu 236 mg des gemäß Beispiel 1 hergestellten Materials (entspr. 0,9 mmol Eisen) werden 10 ml Phytinsäurelösung (23,4 g/l Phytinsäure) zugegeben (Molverhältnis Fe : P = 1 : 0,4). Nach Einstellen des pH-Wertes von 3,0, 5,5 oder 8,0 läßt man die Suspension 2 Stunden bei 37°C reagieren. Danach wird die Suspension zentrifugiert, der Überstand abdekantiert, mit destilliertem Wasser auf 25 ml aufgefüllt und dessen Phosphorgehalt nach Aufschluß des organisch gebundenen Phosphats und Fällung des anorganischen Phosphats mittels Lorenz-Reagens gravimetrisch bestimmt.

**Tabelle 4:**

| Phosphatbindungsvermögen, Molverhältnis Fe : P = 1 : 0,4 | | | | | |
|---|---|---|---|---|---|
| pH | mg Phosphor in der überstehenden Lösung | mg Phosphat in der überstehenden Lösung | mg Phosphor adsorbiert | mg Phosphat adsorbiert | Adsorption in % |
| 3,0 | 2,57 | 6,54 | 8,59 | 21,89 | 77 |
| 5,5 | 3,12 | 7,96 | 8,04 | 20,47 | 72 |
| 8,0 | 3,68 | 9,38 | 7,48 | 19,05 | 67 |

### Beispiel 6

Zu 208,3 g der gemäß Beispiel 1 hergestellten Suspension wird 30,0 g Saccharose zugegeben. Die entstehende Suspension wird sodann am Rotationsverdampfer bei 50°C eingeengt und im Hochvakuum bei 40°C getrocknet. Die Bestimmung der Phosphatbindungskapazität des entstehenden Materials erfolgt analog Beispiel 2:

**Tabelle 5**

| Phosphatbindungsvermögen, Molverhältnis Fe : P = 1 : 0,4 | | | | | |
|---|---|---|---|---|---|
| pH | mg Phosphor in der überstehenden Lösung | mg Phosphat in der überstehenden Lösung | mg Phosphor adsorbiert | mg Phosphat adsorbiert | Adsorption in % |
| 3,0 | - | - | 11,16 | 28,43 | 100 |
| 5,5 | - | - | 11,16 | 28,43 | 100 |
| 8,0 | 1,12 | 2,84 | 10,04 | 25,59 | 90 |

### Beispiel 7

Zu 208,3 g der gemäß Beispiel 1 hergestellten Suspension wird 30,0 g Amylopectin zugegeben. Die entstehende Suspension wird sodann am Rotationsverdampfer bei 50°C eingeengt und im Hochvakuum bei 40°C getrocknet. Die Bestimmung der Phosphatbindungskapazität des entstehenden Materials erfolgt analog Beispiel 2:

**Tabelle 6**

| Phosphatbindungsvermögen, Molverhältnis Fe : P = 1 : 0,4 | | | | | |
|---|---|---|---|---|---|
| pH | mg Phosphor in der überstehenden Lösung | mg Phosphat in der überstehenden Lösung | mg Phosphor adsorbiert | mg Phosphat adsorbiert | Adsorption in % |
| 3,0 5,5 | 3,46 | 8,81 | 7,70 | 19,62 | 69 |
| | 3,91 | 9,95 | 7,25 | 18,48 | 65 |
| 8,0 | 5,36 | 13,65 | 5,80 | 14,78 | 52 |

### Beispiel 8

Zu 208,3 g der gemäß Beispiel 1 hergestellten Suspension wird 30,0 g Weißdextrin (Amylum, Fa. Blattmann) zugegeben. Die entstehende Suspension wird sodann am Rotationsverdampfer bei 50°C eingeengt und im Hochvakuum bei 40°C getrocknet. Die Bestimmung der Phosphatbindungskapazität des entstehenden Materials erfolgt analog Beispiel 2:

**Tabelle 7**

| Phosphatbindungsvermögen, Molverhältnis Fe : P = 1 : 0,4 | | | | | |
|---|---|---|---|---|---|
| pH | mg Phosphor in der überstehenden Lösung | mg Phosphat in der überstehenden Lösung | mg Phosphor adsorbiert | mg Phosphat adsorbiert | Adsorption in % |
| 3,0 | - | - | 11,16 | 28,43 | 100 |
| 5,5 | 0,11 | 0,28 | 11,05 | 28,15 | 99 |
| 8,0 | 2,9 | 7,39 | 8,26 | 21,04 | 74 |

### Beispiel 9

Zu 208,3 g der gemäß Beispiel 1 hergestellten Suspension wird 30,0 g Humussäure (Fluka, Art.-Nr. 53860) zugegeben. Die entstehende Suspension wird sodann am Rotationsverdampfer bei 50°C eingeengt und im Hochvakuum bei 40°C getrocknet. Die Bestimmung der Phosphatbindungskapazität des entstehenden Materials erfolgt analog Beispiel 2:

**Tabelle 8**

| Phosphatbindungsvermögen, Molverhältnis Fe : P = 1 : 0,4 | | | | | |
|---|---|---|---|---|---|
| pH | mg Phosphor in der überstehenden Lösung | mg Phosphat in der überstehenden Lösung | mg Phosphor adsorbiert | mg Phosphat adsorbiert | Adsorption in % |
| 3,0 | 0,89 | 2,27 | 10,27 | 26,16 | 92 |
| 5,5 | 2,46 | 6,25 | 8,70 | 22,18 | 78 |
| 8,0 | 2,79 | 7,11 | 8,37 | 21,32 | 75 |

### Beispiel 10

Bestimmung der Bindungskapazität kommerziell erhältlicher Eisen(III)oxide für anorganisches Phosphat aus einer wäßrigen Phosphatlösung:

Zu der 50 mg Eisen entsprechenden Menge Eisen(III)oxid (entsprechend 0,9 mmol Eisen) werden 10 ml Natriumphosphatlösung (13,68 g/l Na₃PO₄ x 12 H₂O) zugegeben (Molverhältnis Fe : P = 1 : 0,4). Nach Einstellen des pH-Wertes von 3,0, 5,5 oder 8,0 läßt man die Suspension 2 Stunden bei 37°C reagieren. Danach wird die Suspension zentrifugiert, der Überstand abdekantiert, mit destilliertem Wasser auf 25 ml aufgefüllt und dessen Phosphorgehalt mittels eines Phosphor-Molybdän-Tests photometrisch bestimmt.

**Tabelle 9**

| Phosphatbindungsvermögen von Eisen(III)oxid zur Analyse, Fa. Merck, Deutschland, Fe-Gehalt 69,2 %, Molverhältnis Fe : P = 1 : 0,4 | | | | | |
|---|---|---|---|---|---|
| pH | mg Phosphor in der überstehenden Lösung | mg Phosphat in der überstehenden Lösung | mg Phosphor adsorbiert | mg Phosphat adsorbiert | Adsorption in % |
| 3,0 | 10,38 | 26,44 | 0,781 | 1,990 | 7 |
| 5,5 | 10,71 | 27,29 | 0,446 | 1,137 | 4 |
| 8,0 | 10,83 | 27,58 | 0,335 | 0,853 | 3 |

**Tabelle 10**

| Phosphatbindungsvermögen von Eisen(III)oxidmonohydrat, Fa. Strem Chemikals, USA, Fe-Gehalt 61,5 %, Molverhältnis Fe : P = 1 : 0,4 | | | | | |
|---|---|---|---|---|---|
| pH | mg Phosphor in der überstehenden Lösung | mg Phosphat in der überstehenden Lösung | mg Phosphor adsorbiert | mg Phosphat adsorbiert | Adsorption in % |
| 3,0 | 9,71 | 24,73 | 1,451 | 3,696 | 13 |
| 5,5 | 10,49 | 26,72 | 0,670 | 1,706 | 6 |
| 8,0 | 10,38 | 26,44 | 0,781 | 1,990 | 7 |

**Tabelle 11**

| Phosphatbindungsvermögen von Eisen(III)subcarbonat, Fa. Lohmann, Deutschland, Fe-Gehalt 59,3 %, Molverhältnis Fe : P = 1 : 0,4 | | | | | |
|---|---|---|---|---|---|
| pH | mg Phosphor in der überstehenden Lösung | mg Phosphat in der überstehenden Lösung | mg Phosphor adsorbiert | mg Phosphat adsorbiert | Adsorption in % |
| 3,0 | 8,93 | 22,74 | 2,232 | 5,686 | 20 |
| 5,5 | 9,82 | 25,02 | 1,339 | 3,412 | 12 |
| 8,0 | 10,16 | 25,87 | 1,004 | 2,559 | 9 |

### Beispiel 11

Bestimmung der Bindungskapazitäten von α-, β- und γ-Eisenoxyhydroxiden für anorganisches Phosphat aus einer wäßrigen Phosphatlösung. Die Bestimmung der Phosphatbindungskapazität erfolgt analog Beispiel 10:

**Tabelle 12**

| Phosphatbindungsvermögen von α-Eisenoxyhydroxid (Goethit), hergestellt gemäß U. Schwertmann, R. M. Cornell, Iron Oxides in the Laboratory, VCH Weinheim, 64 (1991) Fe-Gehalt 59,7 %, Molverhältnis Fe : P = 1 : 0,4 | | | | | |
|---|---|---|---|---|---|
| pH | mg Phosphor in der überstehenden Lösung | mg Phosphat in der überstehenden Lösung | mg Phosphor adsorbiert | mg Phosphat adsorbiert | Adsorption in % |
| 3,0 | 4,352 | 11,09 | 6,808 | 17,34 | 61 |
| 5,5 | 4,464 | 11,37 | 6,696 | 17,06 | 60 |
| 8,0 | 4,576 | 11,66 | 6,584 | 16,77 | 59 |

**Tabelle 13**

| Phosphatbindungsvermögen von β-Eisenoxyhydroxid (Akaganeit), hergestellt gemäß U. Schwertmann, R. M. Cornell, Iron Oxides in the Laboratory, VCH Weinheim, 95 (1991) Fe-Gehalt 52,2 %, Molverhältnis Fe : P = 1 : 0,4 | | | | | |
|---|---|---|---|---|---|
| pH | mg Phosphor in der überstehenden Lösung | mg Phosphat in der überstehenden Lösung | mg Phosphor adsorbiert | mg Phosphat adsorbiert | Adsorption in % |
| 3,0 | 4,129 | 10,52 | 7,031 | 17,91 | 63 |
| 5,5 | 4,576 | 11,66 | 6,584 | 16,77 | 59 |
| 8,0 | 4,464 | 11,37 | 6,696 | 17,06 | 60 |

**Tabelle 14**

| Phosphatbindungsvermögen von γ-Eisenoxyhydroxid (Lepidocrocit), hergestellt gemäß U. Schwertmann, R. M. Cornell, Iron Oxides in the Laboratory, VCH Weinheim, 81 (1991) Fe-Gehalt 54,4 %, Molverhältnis Fe : P = 1 : 0,4 | | | | | |
|---|---|---|---|---|---|
| pH | mg Phosphor in der überstehenden Lösung | mg Phosphat in der überstehenden Lösung | mg Phosphor adsorbiert | mg Phosphat adsorbiert | Adsorption in % |
| 3,0 | 3,683 | 9,38 | 7,477 | 19,05 | 67 |
| 5,5 | 4,687 | 11,94 | 6,473 | 16,49 | 58 |
| 8,0 | 4,464 | 11,37 | 6,696 | 17,06 | 60 |

### Beispiel 12

Bestimmung der Bindungskapazitäten von stabilisierten α-, β- und γ-Eisenoxyhydroxiden für anorganisches Phosphat aus einer wäßrigen Phosphatlösung. Zu den Eisenoxyhydroxiden aus Beispiel 11 wurden zur Stabilisierung Saccharose und Stärke im Gewichtsverhältnis Fe : Saccharose : Stärke = 1 : 1,5 : 1,5 zugegeben.

Die Bestimmung der Phosphatbindungskapazität erfolgt analog Beispiel 10:

**Tabelle 15:**

| Phosphatbindungsvermögen von stabilisiertem α-Eisenoxyhydroxid (Goethit) Fe-Gehalt 20,6 %, Molverhältnis Fe : P = 1 : 0,4 | | | | | |
|---|---|---|---|---|---|
| pH | mg Phosphor in der überstehenden Lösung | mg Phosphat in der überstehenden Lösung | mg Phosphor adsorbiert | mg Phosphat adsorbiert | Adsorption in % |
| 3,0 | 3,906 | 9,95 | 7,254 | 18,48 | 65 |
| 5,5 | 4,018 | 10,23 | 7,142 | 18,20 | 64 |
| 8,0 | 4,241 | 10,80 | 6,919 | 17,63 | 62 |

**Tabelle 16**

| Phosphatbindungsvermögen von stabilisiertem β-Eisenoxyhydroxid (Akaganeit) Fe-Gehalt 17,7 %, Molverhältnis Fe : P = 1 : 0,4 | | | | | |
|---|---|---|---|---|---|
| pH | mg Phosphor in der überstehenden Lösung | mg Phosphat in der überstehenden Lösung | mg Phosphor adsorbiert | mg Phosphat adsorbiert | Adsorption in % |
| 3,0 | 4,018 | 10,23 | 7,142 | 18,20 | 64 |
| 5,5 | 3,906 | 9,95 | 7,254 | 18,48 | 65 |
| 8,0 | 4,018 | 10,23 | 7,142 | 18,20 | 64 |

**Tabelle 17**

| Phosphatbindungsvermögen von stabilisiertem γ-Eisenoxyhydroxid (Lepiaocrocit), Fe-Gehalt 20,0 %, Molverhältnis Fe : P = 1 : 0,4 | | | | | |
|---|---|---|---|---|---|
| pH | mg Phosphor in der überstehenden Lösung | mg Phosphat in der überstehenden Lösung | mg Phosphor adsorbiert | mg Phosphat adsorbiert | Adsorption in % |
| 3,0 | 3,683 | 9,38 | 7,477 | 19,05 | 67 |
| 5,5 | 2,790 | 7,06 | 8,370 | 21,32 | 75 |
| 8,0 | 3,683 | 9,38 | 7,477 | 19,05 | 67 |

### Beispiel 13

Bestimmung der Bindungskapazität eines Gemisches von Calciumacetat mit dem gemäß Beispiel 1 hergestellten Material für anorganisches Phosphat aus einer wässrigen Phosphatlösung:

Zu 5 g des gemäß Beispiel 1 hergestellten Materials mit einem Eisengehalt von 20,4 % werden 0,96 g Calciumacetat (Gehalt 93,5 - 94,5 %) zugegeben und gut vermischt. Die Bestimmung der Phosphatbindungskapazität des entstehenden Materials erfolgt analog Beispiel 2:

**Tabelle 18**

| Phosphatbindungsvermögen, Molverhältnis Fe : P = 1 : 0,4 | | | | | |
|---|---|---|---|---|---|
| pH | mg Phosphor in der überstehenden Lösung | mg Phosphat in der überstehenden Lösung | mg Phosphor adsorbiert | mg Phosphat adsorbiert | Adsorption in % |
| 3,0 | 1,339 | 3,41 | 9,821 | 25,02 | 88 |
| 5,5 | 1,339 | 3,41 | 9,821 | 25,02 | 88 |
| 8,0 | 1,12 | 2,84 | 10,04 | 25,59 | 90 |

### Beispiel 15

Das gemäß Beispiel 6 hergestellte Material wird mit Wasser in Suspension gebracht, 30 Minuten gerührt, zentrifugiert und die obere Phase abdekantiert. Dies wird solange wiederholt, bis kein Zucker mehr in der wässrigen Phase nachweisbar ist. Auf diese Weise konnten nach dem Auswaschen noch 1,8 % der eingesetzten Saccharose im Präparat nachgewiesen werden. Dies entspricht einem Verhältnis Fe : Saccharose = 15 : 1 im ausgewaschenen Material. Dies zeigt, daß der Zucker nicht komplexgebunden ist.

### Beispiel 16

Zur Herstellung eines Standardrattenfutters mit 0,8 % Gesamtphosphorgehalt wurden die trockenen, pulverförmigen Einzelbestandteile nach intensiver Durchmischung in einen Zylinder gefüllt und zu Pellets gepreßt. Die Zusammensetzung des Futters nach Weender Analyse entsprach den Werten in nachfolgender Tabelle. Dabei verteilte sich der Gesamtphosphorgehalt auf 0,75 % organisch gebundenes und 0,05 % anorganisches Phosphat.

Parallel zu dieser Produktion wurde eine zweite Charge von denselben Ausgangsstoffen und nach gleicher Rezeptur hergestellt, wobei 3 % G/G des gemäß Beispiel 1 hergestellten Materials zusätzlich enthalten waren.

Von beiden Futtermitelchargen wurde nach Abschluß der Produktion und einer Lagerzeit von ca. 2 Monaten eine Analyse auf verfügbares Phosphat durchgeführt. Dabei konnte in der Kontrolle des nicht phosphatadsorberhaltigen Produktes ein Phosphatgehalt von 0,68 % entsprechend 84,5 % der eingesetzten Menge und im Prüfpräparat 0,50 % entsprechend 62,6 % der eingesetzen Menge wiedergefunden werden.

Die Differenz von 21,9 % entsprechend 1,75 mg P/g Futter entspricht bei 3 % Adsorbergehalt einem Bindungsvermögen von 1,9 mmol P/g Adsorber.

Ergebnis der Weender Futtermittelanalyse für das Kontrollpräparat ohne Adsorber:

| | |
|---|---|
| Rohprotein | 19,7 % |
| Rohfett | 3,2 % |
| Rohfaser | 5,4 % |
| Rohasche | 6,5 % |
| Wasser | 10,9 % |
| N - freie Extr. | 54,3 % |
| Ca - Gehalt | 1,2 % |
| P - Gehalt | 0,8 % |
| Pellethärte nach Kahl | 17,3 |

## Patentansprüche

1. Adsorbens für Phosphat aus wäßrigem Medium, das durch Kohlenhydrate und/oder Humussäure stabilisiertes polynukleares beta-Eisenhydroxid enthält.

2. Verfahren zur Herstellung des Absorbens nach Anspruch 1, **dadurch gekennzeichnet, daß** man eine wäßrige Basenlösung mit einer wäßrigen, Chloridionen enthaltenden Lösung von Eisen-III-salz unter Bildung einer Suspension mit einem pH-Wert von 3 bis 10 vermischt, die Suspension ruhen läßt, Waschen der erhaltenen Ausfällung mit Wasser, Suspendieren der noch feuchten Ausfällung in Wasser unter Bildung einer Suspension mit einem Eisengehalt von bis zu 6 Gew.-% und Zugabe von einem oder mehreren Kohlenhydraten und/oder Humussäure in einer derartigen Menge, daß der erhaltene Feststoff maximal 40 Gew.-% Eisen enthält.

3. Verfahren nach Anspruch 2, **dadurch gekennzeichnet, daß** man eine wäßrige Alkalicarbonat- oder Alkalibicarbonatlösung mit einer wäßrigen, Chloridionen enthaltenden Lösung von Eisen-III-salz unter Bildung einer Suspension mit einem pH-Wert von über 6 vermischt, die Suspension ruhen läßt, Waschen der erhaltenen Ausfällung mit Wasser zur Entfernung der vorhandenen Chloridionen, Suspendieren der noch feuchten Ausfällung in Wasser unter Bildung einer Suspension mit einem Eisengehalt von bis zu 6 Gew.-% und Zugabe von einem oder mehreren Kohlenhydraten und/oder Humussäure in einer derartigen Menge, daß der erhaltene Feststoff maximal 40 Gew.-% Eisen enthält.

4. Verfahren nach Anspruch 2 oder 3, **dadurch gekennzeichnet, daß** als Eisen-III-salz Eisen-III-Chlorid verwendet wird.

5. Verfahren nach Anspruch 2, 3 oder 4, **dadurch gekennzeichnet, daß** als Alkalicarbonat-, Alkalibicarbonat- und/oder Base Natriumcarbonat oder Natriumbicarbonat verwendet wird.

6. Adsorbens nach Anspruch 1, **dadurch gekennzeichnet, daß** das stabilisierte polynukleare beta-Eisenhydroxid erhältlich ist nach dem Verfahren von Anspruch 2, 3, 4 oder 5.

7. Adsorbens nach einem der Ansprüche 1 oder 6, **dadurch gekennzeichnet, daß** das Kohlenhydrat Saccharose, Dextrin oder eine Mischung davon ist.

8. Adsorbens nach einem der Ansprüche 1, 6 oder 7, **dadurch gekennzeichnet, daß** es Calciumsalz enthält.

9. Verfahren nach einem der Ansprüche 3 bis 5, **dadurch gekennzeichnet, daß** als Kohlenhydrat Saccharose, Dextrin oder eine Mischung davon eingesetzt wird.

10. Verfahren nach einem der Ansprüche 3 bis 5 oder 9, **dadurch gekennzeichnet, daß** ein Calciumsalz zugesetzt wird.

11. Verwendung des Adsorbens nach einem der Ansprüche 1 und 6 bis 8 zur Adsorption von Phosphat aus wäßrigem Medium, insbesondere aus wäßrigen Lösungen.

12. Verwendung des Adsorbens nach einem der Ansprüche 1 und 6 bis 8 zur Adsorption von anorganischen und nahrungsmittelgebundenem Phosphat aus Nahrungsmitteln.

13. Verwendung von durch Kohlenhydrate und/oder Humussäure stabilisiertem polynuklearem beta-Eisenhydroxid, wie in einem der Ansprüche 1 und 6 bis 8 definiert, zur Herstellung von Adsorbentien zur Beimischung zu Nahrungsmitteln.

## Claims

1. An adsorbent for phosphate from an aqueous medium, which adsorbent contains polynuclear beta-iron hydroxide stabilised by carbohydrates and/or humic acid.

2. A method of producing an adsorbent according to claim 1, **characterised in that** an aqueous solution of a base is mixed with an aqueous solution of an iron(III) salt, which contains chloride ions, to form a suspension with a pH of 3 to 10, the suspension is allowed to stand, the precipitate obtained is washed with water, the still moist precipitate is suspended in water to form a suspension with an iron content of up to 6 % by weight, and one or more carbohydrates and/or humic acid are added in an amount such that the solid obtained contains a maximum of 40 % by weight of iron.

3. A method according to claim 2, **characterised in that** an aqueous solution of an alkali carbonate or alkali bicarbonate is mixed with an aqueous solution of an iron(III) salt, which contains chloride ions, to form a suspension with a pH greater than 6, the suspension is allowed to stand, the precipitate obtained is washed with water to remove the chloride ions which are present, the still moist precipitate is suspended in water to form a suspension with an iron content of up to 6 % by weight, and one or more carbohydrates and/or humic acid are added in an amount such a that the solid contains a maximum of 40 % by weight of iron.

4. A method according to claims 2 or 3, **characterised in that** iron(III) chloride is used as an iron(III) salt.

5. A method according to claims 2, 3 or 4, **characterised in that** sodium carbonate or sodium bicarbonate is used as an alkali carbonate, alkali bicarbonate and/or base.

6. An absorbent according to claim 1, **characterised in that** the stabilised polynuclear beta-iron hydroxide can be obtained by the method of claims 2, 3, 4 or 5.

7. An adsorbent according to either one of claims 1 or 6, **characterised in that** the carbohydrate is saccharose, dextrin or a mixture thereof.

8. An absorbent according to any one of claims 1, 6 or 7, **characterised in that** it contains a calcium salt.

9. A method according to any one of claims 3 to 5, **characterised in that** saccharose, dextrin or a mixture thereof is used as a carbohydrate.

10. A method according to any one of claims 3 to 5 or 9, **characterised in that** a calcium salt is added.

11. Use of the absorbent according to any one of claims 1 and 6 to 8 for the absorption of phosphate from an aqueous medium, particularly from aqueous solutions.

12. Use of the adsorbent according to any one of claims 1 and 6 to 8 for the absorption of inorganic and nutrient-bound phosphate from nutrients.

13. Use of polynuclear beta-iron hydroxide which is stabilised by carbohydrates and/or humic acid, as defined in any one of claims 1 and 6 to 8, for the production of absorbents for admixture with nutrients.

## Revendications

1. Adsorbant pour du phosphate formé à partir d'un milieu aqueux contenant de l'hydroxyde de fer bêta polynucléaire stabilisé par des hydrates de carbone et/ou de l'acide humique.

2. Procédé de fabrication de l'adsorbant selon la revendication 1, **caractérisé en ce que** l'on mélange une solution aqueuse de base avec une solution aqueuse de fer III contenant des ions chlorures avec formation d'une suspension à un pH de 3 à 10, on laisse reposer la suspension, on lave le précipité obtenu avec de l'eau, on met le précipité encore humide en suspension dans de l'eau avec formation d'une suspension ayant une teneur en fer allant jusqu'à 6% en poids et on ajoute un ou plusieurs hydrates de carbone et/ou de l'acide humique en une quantité telle que le solide obtenu contienne au maximum 40% en poids de fer.

3. Procédé selon la revendication 2, **caractérisé en ce que** l'on mélange une solution aqueuse de carbonate ou bicarbonate alcalin avec une solution aqueuse de fer III contenant des ions chlorures avec formation d'une suspension à un pH supérieur à 6, on laisse reposer la suspension, on lave le précipité obtenu avec de l'eau, on met le précipité encore humide en suspension dans de l'eau avec formation d'une suspension ayant une teneur en fer allant jusqu'à 6% en poids et on ajoute un ou plusieurs hydrates de carbone et/ou de l'acide humique en une quantité telle que le solide obtenu contienne au maximum 40% en poids de fer.

4. Procédé selon la revendication 2 ou la revendication 3, **caractérisé en ce que** le sel de fer III utilisé est le chlorure de fer III.

5. Procédé selon la revendication 2, la revendication 3 ou la revendication 4, **caractérisé en ce que** le carbonate ou bicarbonate alcalin et/ou la base est le carbonate de sodium ou le bicarbonate de sodium.

6. Adsorbant selon la revendication 1, **caractérisé en ce que** l'hydroxyde de fer bêta polynucléaire stabilisé peut être obtenu par le procédé selon la revendication 2, 3, 4 ou 5.

7. Adsorbant selon l'une des revendications 1 ou 6, **caractérisé en ce que** l'hydrate de carbone est le saccharose, la dextrine ou un mélange de ceux-ci.

8. Adsorbant selon l'une des revendications 1, 6 ou 7, **caractérisé en ce qu'**il contient du sel de calcium.

9. Procédé selon l'une des revendications 3 à 5, **caractérisé en ce que** l'on utilise comme hydrate de carbone du saccharose, de la dextrine ou un mélange de ceux-ci.

10. Procédé selon l'une des revendications 3 à 5 ou 9, **caractérisé en ce que** l'on ajoute un sel de calcium

11. Utilisation de l'adsorbant selon l'une des revendications 1 et 6 à 8 pour l'adsorption de phosphate à partir d'un milieu aqueux, en particulier à partir de solutions aqueuses.

12. Utilisation de l'adsorbant selon l'une des revendications 1 et 6 à 8 pour l'adsorption de phosphate inorganique et lié aux aliments à partir de produits alimentaires.

13. Utilisation d'hydroxyde de fer bêta polynucléaire stabilisé par des hydrates de carbone et/ou de l'acide humique tel que défini dans les revendications 1 et 6 à 8 pour la fabrication d'adsorbants destinés à être incorporés dans des produits alimentaires.
